Europäisches Patentamt

(19) European Patent Office

Office européen des brevets

(11) Publication number: **0 064 289**
**B2**

(12) NEW EUROPEAN PATENT SPECIFICATION

(45) Date of publication of the new patent
specification: 02.05.90

(21) Application number: 82103777.7

(22) Date of filing: 03.05.82

(51) Int. Cl.⁵: **A 61 N 1/05**

(54) Body implantable lead.

㉚ Priorïty: 04.05.81 US 260532

(43) Date of publication of application:
10.11.82 Bulletin 82/45

(45) Publication of the grant of the patent:
26.03.86 Bulletin 86/13

(45) Mention of the opposition decision:
02.05.90 Bulletin 90/18

(84) Designated Contracting States:
DE FR GB IT NL SE

(56) References cited:
EP-A-0 043 461    US-A-3 749 101
EP-A-0 054 781    US-A-3 813 258
DE-A-1 571 721

IEEE INTERNATIONAL CONVENTION RECORD,
vol. 15, no. 9, 20th-23rd March, 1967, pages
109-117, New York, USA, W. GREATBATCH:
"The electrode interface between man and a
prosthetic stimulator"

(73) Proprietor: **MEDTRONIC, INC.**
**3055 Old Highway Eight**
**Minneapolis Minnesota 55440 (US)**

(72) Inventor: **Kraska, Robert C.**
**3151 Garfield Street N.E.**
**Minneapolis Minn. 55418 (US)**
Inventor: **Lessar, Joseph F.**
**3742 114th Lane N.W.**
**Coon Rapids, Minn. 55433 (US)**

(74) Representative: **Schwan, Gerhard, Dipl.-Ing.**
**Elfenstrasse 32**
**D-8000 München 83 (DE)**

# EP 0 064 289 B1

(56) References cited:

MEDICAL AND BIOLOGICAL ENGINEERING, vol. 10, no. 10, September 1972, pages 662-672, Stevenage, G.B. E.G. MERRILL et al.: "Glass-coated platinum-plated tungsten microelectrodes"

THIN SOLID FILMS, vol. 63, no. 1, October 1979, pages 55-60, Lausanne, CH., S. FUJISHIRO et al.: "Creep property improvement of alpha-beta titanium alloys by platinum ion plating"

MEDICAL AND BIOLOGICAL ENGINEERING, vol. 12, no. 6, November 1974, pages 778-790, Stevenage, G.B., M. SONN et al.: "A prototype flexible microelectrode array for implant-prosthesis applications"

## Description

The present invention relates to a body implantable lead.

It is common to use platinum and platinum iridium electrodes in body implantable leads for cardiac pacing. This is desirable because the platinum and platinum iridium alloy (usually 90% platinum and 10% iridium) have a high resistance to corrosion within the implant environment. These materials are also desirable for the construction of electrodes because of the relatively low polarization associated with electrodes from this material. The major disadvantage to this technique, however, is that a solid platinum or platinum iridium electrode is quite expensive to fabricate because of the high raw material cost.

A second approach to electrode fabrication is discussed by Williamson in US—A—3 749 101.

Williamson describes an electrode having a basic titantium structure with a platinum insert. Williamson teaches that by platinizing (i.e., plating platinum onto) the platinum insert to produce platinum black, a non-polarizing muscle stimulating electrode is fabricated. This technique, though not in and of itself popularly used, does teach the coating of a titanium electrode housing with a layer of a noble metal, in this case platinum. Notice, however, in the teaching of Williamson, the titanium is not totally covered and therefore is also available as a stimulation surface. This factor is brought out very clearly at column 4, lines 5—7 in which Williamson states that his lead "features a second electrode housing which is itself capable of carrying the stimulating current should there by any malfunction of the platinum."

A further problem that is found in Williamson is the difficulty in attaching the platinum sleeve or coil reliably to the titanium electrode assembly. Williamson does not go into any great detail in this matter, but those of ordinary skill in the art will be able to see that there is some difficulty in establishing and maintaining reliable attachment over several years' use. It is well known in the scientific community that platinum black is very fragile such that in a cardiac electrode application, it would be susceptible to detachment simply by insertion into the heart. It is also established that platinum black is very susceptible to chemical contamination which would tend to negate its non-polarization properties.

A topic not addressed by Williamson is that other metals within the platinum group (i.e., rhodium and iridium) may in fact be more desirable as electrode surfaces than is platinum or an alloy of 90% platinum and 10% iridium. Little experimentation has been conducted with these materials since they, in fact, are too hard to be easily manufactured into electrodes of implantable size using techniques conventionally used to manufacture electrodes.

Furthermore, EP—A—0 043 461 describes a body implantable cardiac stimulator lead having an exposed electrode tip, which tip comprises a body of a platinum iridium alloy electrolytically coated with platinum sponge the porous structure of which is consolidated by sintering. EP—A— 0 043 461 is part of the prior art under Article 54 (3) and (4) EPC and thus relevant only when evaluating the novelty of the present invention with respect to Designated States DE, FR, GB and NL.

Besides, a flexible microelectrode array for implant-prosthesis applications is known (Medical and Biological Engineering, Nov. 1974, pages 778—790), which microelectrode array comprises a layer of platinum deposited by sputtering on a plastic film substrate.

An object of the present invention is to provide for a body implantable lead which allows reducing the raw material cost of the electrode.

In conformity with the present invention a body implantable lead for delivery of stimulation energy to a desired body site of the type having an exposed electrode carried by the lead, said electrode comprising an electrode assembly of a body compatible first material and a second material covering at least partly the exposed portion of said first material, said second material being a noble metal or an alloy thereof, is characterized in that the whole exposed portion of said first material is covered by a sputtered intermediate layer of a reactive metal and by said second material which consists of a further sputtered layer of a noble metal or an alloy thereof sputtered over said sputtered intermediate layer.

In the case of the lead of the present invention a very tiny amount of precious metal is placed in a very thin layer over the top of a base electrode assembly. This technique is very important in reducing the raw material cost of the electrode assembly. The second major advantage of the present invention is that the sputtering of the surface material over a sputtered intermediate layer, preferably titanium, which is itself sputtered over a base metal, provides reliable adhesion of the noble metal electrode surface. A third important and interesting aspect of the present invention is that it permits use of materials for the electrode surface which are not currently useful in the prior art electrodes. This is because the basic shape and characteristic form of the electrode assembly are created by common manufacturing techniques applied to the base metal electrode assembly. The material used for the stimulating surface, because it is merely sputtered on, need not be machined or worked in any way to establish its basic form. The basic form is established by the base metal electrode assembly. For this reason, materials having very desirable corrosion resistance and low polarization properties such as rhodium and iridium may now be used on body implantable leads without the difficulties associated with machining and forming such materials.

Fig. 1 is a plan view of a body implantable lead of the unipolar type incorporating the present invention.

Fig. 2 is a cross sectional view of the electrode assembly employing the present invention incorporated within the body implantable lead of Fig. 1.

The present invention is described in relation to the preferred mode of its practice. This involves the construction of a unipolar tined lead for stimulation and sensing using a cardiac pacer within the right ventricle. Those of ordinary skill in the art however, will be readily able to apply the invention as taught herein to other types of body implantable leads.

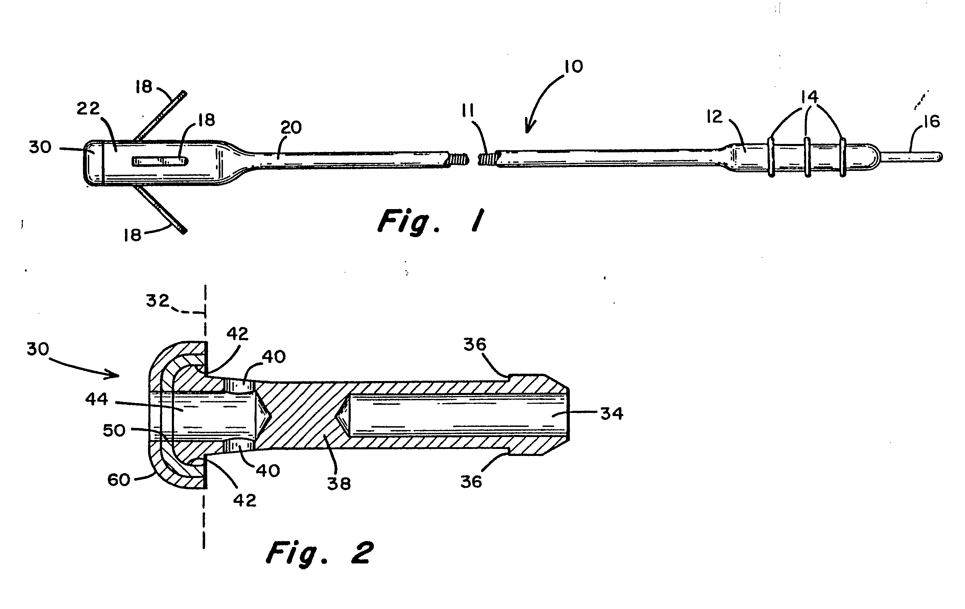

Fig. 1 is a plan view of a body implantable lead 10 incorporating the present invention. At the proximal end of the body implantable lead 10, a conductive terminal pin 16, which is attached to and used to conduct a stimulation pulse from an implantable pulse generator (not shown) to the body implantable lead, is attached to a length of coiled wire conductor 11. An insulator sheath 12 is formed about a portion of the pin 16 and the electrical conductor 11. O-rings 14 formed from the insulator 12 are used to establish a proper seal between connector sleeve 12 and the implantable pulse generator to prevent the ingress of body fluids.

The conductor 11 within the main body of body implantable lead 10 is covered with an insulating sheath 20. Insulating sheath 20 is preferably of polyurethane material. However, other commonly used body compatible insulators such as silicone rubber may be used. Insulating sheath 20 is stretched or formed at its distal end to cover the entire electrode assembly at enlargement 22.

Non-conducting tines 18 are employed to assist in acute fixation in the manner common in the art. The stimulating surface of body implantable lead 10 is at the very distal tip. The distal tip 30 directly contacts body stimulatable tissue during pacing. The lead as described is similar to that shown and described in US—A—3 902 501.

Fig. 2 is side sectional view of the electrode employed in body implantable lead 10 of the present invention. Imaginary line 32 has been drawn to show the extent to which distal tip 30 is exposed. That is, that part of the electrode assembly which is proximal (to the right) of imaginary line 32 is covered by the stretched portion 22 of outer sheath 20 (shown in Fig. 1). That portion of the electrode assembly in Fig. 2 which is distal of dashed line 32 is the exposed distal tip 30.

The electrode assembly has a longitudinal lumen 34 into which the conductor coil 11 of the body implantable lead is inserted. This body implantable lead coil 11 is attached, using methods common in the art in the manner shown, for example, in the above-referenced patents. Shoulders 36 are used to ensure that outer sheath 20 is properly gripped by the electrode assembly to ensure that the outer sheath 20 is reliably attached. The entire electrode assembly 38 is frabricated from a single piece of commercially pure grade titanium.

A distal lumen 44 is used for the insertion of a round piece of insulating material. This material reduces the overall effective stimulating surface and thereby increases effective current density of the electrode. Tranvserse lumen 40 is used to attach the non-conducting material which is inserted into distal lumen 44.

Layer 50 is a layer of reactive metal e.g., titanium, niobium, etc.) which is sputtered over the distal tip of titanium electrode assembly 38. This layer is on the order of 100 to 1,000 nm in thickness for the preferred titanium although thicknesses of as much as 5,000 nm are readily achievable in relatively short sputtering cycles. This layer of sputtered titanium (or other reactive metal) coats surface 42 of titanium electrode assembly 38. The sputtered layer 50 adheres to surface 42 of titanium electrode assembly 38, because a material is chosen which exhibits high chemical reactivity. In the preferred mode, this coincidentally produces a titanium/titanium interface at surface 42. However, sputtered layer 50 may be niobium or other highly reactive metal.

A layer of precious metal 60 is next sputtered over the sputtered titanium layer 50. The thickness of layer 60 may be as much as 5,000 nm although a thickness of 100 nm to 1,000 nm is preferred. Because layer 60 is sputtered over layer 50, and layer 50 has been activated by the previous sputtering operation, excellent adhesion is established between previous metal layer 60 and reactive metal layer 50. Layer 60 may be selected from the gorup comprising platinum, rhodium, iridium, or alloys thereof.

Of course, Fig. 2 shows layers 50 and 60 much larger than scale. However, they are shown in this manner for the purpose of visualizing the present invention.

The sputtering process is accomplished using a Perkin-Elmer Model 2400 Sputtering System. This device has the capability for both AC and Magnetron modes. Experience has shown that the Magnetron mode produces a higher sputtering rate, being on the order of 1,000 to 10,000 nm per hour because of the magnetic field present. However, the AC mode which has a slower sputtering rate is preferred because it produces much more uniform target ware.

Although the controlling of pressure within the sputtering chamber controls porosity and therefore, the presence of pinholes, the resulting product will almost always contain some significant number of pinholes. It is therefore very important to use a material for electrode housing 38, which is of itself a body compatible cathode material such as titanium.

Attempts were made to fabricate similar electrodes using vacuum deposition techniques. Because the target in the vacuum deposition process is not self-cleaning as it is in the sputtering process, results obtained were not nearly as satisfying. The vacuum deposition electrodes showed less adhesion because the target was not self-cleaning and because the technique inherently provides less control over the porosity of the surface.

The present invention as taught herein is readily applied in the preferred mode to endocardial pacing electrodes. Those of ordinary skill in the art will be readily able to apply the teachings herein to body implantable leads of other configurations and applications.

## Claims

1. body implantable lead (10) for delivery of stimulation energy to a desired body site of the type having an exposed electrode (30) carried by the lead, said electrode comprising an electrode assembly (38) of a body implantable first material and a second material covering at least partly the exposed portion of said first material, said second material being a noble metal or an alloy thereof, characterized in that the whole exposed portion of said first material is covered by a sputtered intermediate layer (50) of a reactive metal and by said second material which consists of a further sputtered layer (60) of a noble metal or an alloy thereof sputtered over said sputtered intermediate layer (50).

2. A body implantable lead according to claim 1 wherein said further sputtered layer (60) has a thickness no greater than 5,000 nm.

3. A body implantable lead according to claim 2 wherein said further sputtered layer (60) has a thickness of approximately 100 to 1,000 nm.

4. A body implantable lead according to claim 1, 2 or 3 wherein said first material is titanium.

5. A body implantable lead according to any one of claims 1 to 4 wherein said second material is selected from the group consisting of platinum, iridium, rhodium and alloys thereof.

6. A body implantable lead according to any one of the preceding claims wherein said sputtered intermediate layer (50) is 100 nm to 1,000 nm in thickness.

7. A body implantable lead according to any one of the preceding claims wherein said sputtered intermediate layer (50) consists of titanium.

## Patentansprüche

1. In den Körper implantierbare Leitung (10) zur Beaufschlagung einer gewünschten Körperstelle mit Stimulationsenergie, mit einer von der Leitung getragenen freiliegenden Elektrode (30), die eine Elektrodenanordnung (38) aus einem körperkompatiblen ersten Werkstoff und einen zweiten Werkstoff aufweist, der den freiliegenden Teil des ersten Werkstoffes mindestens teilweise abdeckt, wobei der zweite Werkstoff ein Edelmetall oder eine Edelmetallegierung ist, dadurch gekennzeichnet, daß der gesamte freiliegende Teil des ersten Werkstoffes mit einer gesputterten Zwischenschicht (50) aus einem reaktionsfähigen Metall und mit dem zweiten Werkstoff abgedeckt ist, der aus einer weiteren gesputterten Schicht (60) aus einem Edelmetall oder einer Edelmetallegierung besteht, die über die gesputterte Zwischenschicht (50) gesputtert ist.

2. In den Körper implantierbare Leitung nach Anspruch 1, wobei die weitere gesputterte Schicht (60) eine Dicke von nicht mehr als 5.000 nm hat.

3. In den Körper implantierbare Leitung nach Anspruch 2, wobei die weitere gesputterte Schicht (60) eine Dicke von näherungsweise 100 bis 1.000 nm hat.

4. In den Körper implantierbare Leitung nach Anspruch 1, 2 oder 3, wobei der erste Werkstoff Titan ist.

5. In den Körper implantierbare Leitung nach einem der Ansprüche 1 bis 4, wobei der zweite Werkstoff aus der aus Platin, Iridium, Rhodium und deren Legierungen bestehenden Gruppe ausgewählt ist.

6. In den Körper implantierbare Leitung nach einem der vorhergehenden Ansprüche, wobei die gesputterte Zwischenschicht (50) eine Dicke von 100 nm bis 1.000 nm hat.

7. In den Körper implantierbare Leitung nach einem der vorhergehenden Ansprüche, wobei die gesputterte Zwischenschicht (50) aus Titan besteht.

## Revendications

1. Conducteur (10) implantable dans le corps destiné à délivrer de l'énergie de stimulation à une région voulue du corps, du type comprenant une électrode exposée (30) supportée par le conducteur, ladite électrode comprenant un ensemble d'électrode (38) d'une première matière compatible avec le corps et d'une second matière recouvrant au moins partiellement la partie exposée de ladite première matière, ladite seconde matière étant un métal noble ou l'un de ses alliages, caractérisé en ce que toute la partie de ladite première matière est recouverte par une couche intermédiare pulvérisée d'un métal réactif et par ladite seconde matière qui consiste en une couche pulvérisée supplémentaire (60) d'un métal noble ou de l'un de ses alliages pulvérisé sur ladite couche intermédiare pulvérisée.

2. Conducteur implantable dans le corps selon la revendication 1, dans lequel ladite couche pulvérisée supplémentaire (60) a une épaisseur qui ne dépasse par 5000 nm.

3. Conducteur implantable dans le corps selon la revendication 2, dans lequel ladite couche pulvérisée (60) a une épaisseur d'environ 100 à 1000 nm.

4. Conducteur implantable dans le corps selon la revendication 1, 2 ou 3, dans lequel ladite première matière est du titane.

5. Conducteur implantable dans le corps selon l'une quelconque des revendications 1 à 4, dans lequel ladite second matière est choisie dans le groupe comprenant le platine, l'iridium, le rhodium et leurs alliages.

6. Conducteur implantable dans le corps selon l'une quelconque des revendications précédentes, caractérisée en ce que ladite couche intermédiaire pulvérisée (50) a une épaisseur de 100 nm à 1000 nm.

7. Conducteur implantable dans le corps selon l'une quelconque des revendications précédentes, dans lequel ladite couche intermédiaire pulvérisée (50) est constituée de titane.

Fig. 1

Fig. 2

EP 0 064 289 B1